(19) Europäisches Patentamt
European Patent Office
Office européen des brevets

(11) Veröffentlichungsnummer: **0 065 196**
**A1**

(12) # EUROPÄISCHE PATENTANMELDUNG

(21) Anmeldenummer: **82103841.1**

(51) Int. Cl.³: **A 01 N 43/32**

(22) Anmeldetag: **05.05.82**

(30) Priorität: **14.05.81 DE 3119200**

(43) Veröffentlichungstag der Anmeldung: **24.11.82**
**Patentblatt 82/47**

(84) Benannte Vertragsstaaten: **AT BE CH DE FR GB IT LI**

(71) Anmelder: **BASF Aktiengesellschaft,**
**Carl-Bosch-Strasse 38, D-6700 Ludwigshafen (DE)**

(72) Erfinder: **Feuerherd, Karl-Heinz, Dr., c/o BASF Japan Ltd. C.P.O. Box 1757, Tokyo 100-91 (JP)**
Erfinder: **Rentzea, Costin, Dr., Neuenheimer Landstrasse 72, D-6900 Heidelberg (DE)**
Erfinder: **Goetz, Norbert, Dr., Schoefferstrasse 25, D-6520 Worms (DE)**
Erfinder: **Wuerzer, Bruno, Dr. Dipl.-Landwirt, Ruedigerstrasse 13, D-6701 Otterstadt (DE)**

(54) **Herbizides Mittel und dessen Verwendung.**

(57) Die vorliegende Erfindung betrifft herbizide Mittel, die eine Verbindung der Formel

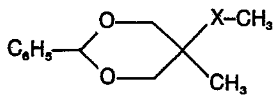
,

worin X eine CO- oder CHOH-Gruppe darstellt, enthalten, sowie die Verwendung dieser Substanzen zur Bekämpfung unerwünschten Pflanzenwuchses.

EP 0 065 196 A1

## Herbizides Mittel und dessen Verwendung

Die vorliegende Erfindung betrifft ein neues herbizides Mittel, welches ein substituiertes 1,3-Dioxan enthält, sowie dessen Verwendung zur Bekämpfung unerwünschten Pflanzenwuchses.

Aus Tetrahedron 26 693 (1970) sind bereits Verbindungen bekannt, die die Formel I

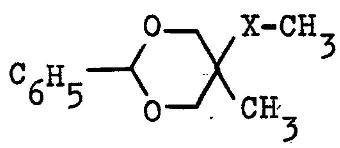

I,

besitzen, in der X eine CO- oder CHOH-Gruppe darstellt. Von diesen Verbindungen ist jedoch nichts über irgendwelche biologischen Wirkungen bekannt.

Es wurde nun gefunden, daß die genannten Verbindungen der Formel I in für Kulturpflanzen selektiver Weise eine Reihe von unerwünschten Pflanzen bekämpfen.

Die Verbindungen der Formel I lassen sich herstellen, indem man

A. 2-Acetyl-2-methyl-1,3-dihydroxypropan mit

a) Benzaldehyd oder
b) einem Benzaldehydacetal der Formel II

$$C_6H_5-CH(OR)_2,$$

worin R Methyl, Ethyl oder beide Reste R zusammen Ethylen bedeuten, oder

c) Benzaldehyd-diacetat oder

d) Benzylidenchlorid in Gegenwart eines Lösungs- oder Verdünnungsmittels und gegebenenfalls unter Zusatz einer anorganischen oder organischen Säure, bei Temperaturen zwischen 20 und 150°C gegebenenfalls unter azeotropischer Auskreisung des Reaktionswassers kondensiert, oder

B. Methylethylketon mit

a) einer 10 bis 42 %igen, wäßrigen Formaldehydlösung oder
b) Paraformaldehyd oder
c) Dimethoxyethan oder
d) Trioxan und Benzaldehyd oder Benzaldehyd-Dimethyl- bzw. Diethylacetal oder Benzaldehyd-Diacetat oder Benzylidendichlorid gegebenenfalls in Gegenwart eines Lösungs- oder Verdünnungsmittels und gegebenenfalls unter Zusatz einer anorganischen oder organischen Base und/oder einer anorganischen oder organischen Säure, bei Temperaturen zwischen 20 und 150°C gegebenenfalls unter azeotropischer Auskreisung des Reaktionswassers kondensiert und das so erhaltene Keton I (X = CO) gegebenenfalls anschliessend nach üblichen Methoden zum Alkohol I (X = CH(OH)) reduziert.

Zu den bevorzugten Lösungs- und Verdünnungsmitteln für das Verfahren A) und B) gehören Wasser, Alkohole wie Methanol, Ethanol, Isopropanol und Butanol, Kohlenwasserstoffe wie n-Hexan, n-Heptan, n- und iso-Octan, Cyclohexan, Tetrahydronaphthalin, Benzol, Toluol, Xylol, Cumol; halogenierte Kohlenwasserstoffe wie Methylenchlorid, Chloroform, Tetrachlorkohlenstoff, 1,2-Dichlorethan und Chlorbenzol; Ether wie Diethylether, Dibutylether, Tetrahydro-

Turan, 1,4-Dioxan, 1,2-Dimethoxyethan und Anisol; Amide wie Dimethylformamid und Dimethylacetamid oder entsprechende Gemische.

Geeignete Basen und Säuren, die gegebenenfalls in katalytischen bis zu stöchiometrischen Mengen verwendet werden, sind beispielsweise Alkali- und Erdalkalihydroxide, wie Natrium-, Kalium-, Calcium- und Bariumhydroxid, Alkoxide wie Natrium- und Kaliummethoxid, -ethoxid, -isopropoxid und tert.-butoxid, Amine wie Triethylamin, N,N-Dimethylcyclohexylamin oder Pyridin, ferner anorganische Säuren wie Salzsäure, Phosphorsäure und Schwefelsäure oder organische Säuren wie Ameisensäure, p-Toluolsulfonsäure, Dodecylbenzolsulfonsäure oder Trifluormethansulfonsäure.

Bevorzugte Konfiguration der erfindungsgemäßen herbiziden Verbindungen ist diejenige worin die Phenylgruppe und die Gruppe $-X-CH_3$ eine cis-Stellung einnehmen. Die oben angegebene cis-Konfiguration tritt dann auf, wenn die Phenylgruppe in äquatorialer Stellung und die Gruppe $-X-CH_3$ in axialer Stellung stehen.

Bei der Verwendung der erfindungsgemäßen Verbindungen als Herbizide kann man die aktiveren cis-Isomeren zusammen mit den trans-Isomeren verwenden und die Mischungen können sogar einen überwiegenden Anteil der letzteren Isomeren enthalten. Je höher der Gehalt an cis-Isomeren ist, um so höher ist die herbizide Wirkung einer gegebenen Mischung an cis- und trans-Isomeren. Bevorzugt liegt das cis:trans--Verhältnis höher als 3:1 und noch bevorzugter höher als 4:1.

Die folgenden Beispiele 1 und 2 erläutern die Herstellung der Wirkstoffe.

Beispiel 1

Die Lösung von 72,6 g (1,0 Mol) Methylethylketon und 1 g Lithiumhydroxid in 440 ml Wasser wird mit 200 g (2,0 Mol) einer 30 %igen wäßrigen Formaldehydlösung tropfenweise versetzt und 12 Stunden nachgerührt. Nach Zugabe von 15 g p-Toluolsulfonsäurehydrat, 800 ml Toluol und 128 g (1,2 Mol) Benzaldehyd wird das Gemisch auf 115°C erwärmt und das Wasser azeotropisch ausgekreist. Nach Abkühlen auf 20°C werden nacheinander 100 ml 35 %ige Natriumhydrogensulfitlösung und 500 ml Wasser zugegeben, 30 Minuten nachgerührt und die organische Phase abgetrennt, getrocknet und eingeengt. Der feste Rückstand wird mit 60 ml Ethanol bei +5°C dispergiert und abgesaugt. Man erhält 107,7 g (49 % d.Th.) 2-Phenyl-5-acetyl-5-methyl-1,3-dioxan vom Schmelzpunkt 115-118°C.

Beispiel 2

Die Lösung von 88 g (0,4 Mol) 2-Phenyl-5-acetyl-5-methyl-1,3-dioxan in 600 ml Methanol wird bei 0 bis -5°C portionsweise mit 18,6 g (0,49 Mol) Natriumborhydrid versetzt, anschließend 1 Stunde bei 0°C und weitere 12 Stunden bei 20°C nachgerührt und im Vakuum eingeengt. Der Rückstand wird mit 500 ml Methylenchlorid und 400 ml 10 %iger Kalilauge 30 Minuten gerührt, die organische Schicht abgetrennt, getrocknet und eingeengt. Der farblose, kristalline Rückstand wird mit 40 ml Ether bei +5°C dispergiert und abgesaugt. Man erhält 60,4 g (68 % d.Th.) 2-Phenyl-5-(1'-hydroxyethyl)-5-methyl-1,3-dioxan vom Schmelzpunkt 61°C.

Die Anwendung der Wirkstoffe erfolgt z.B. in Form von direkt versprühbaren Lösungen, Pulvern, Suspensionen - auch hochprozentige wässerige, ölige oder sonstige Suspensionen - oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem mit hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier-oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:
Alkali-, Erdalkali-, Ammoniumsalze von Ligninsulfonsäure,

Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die Wirkstoffe werden angewendet, beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

Beispiel I

Man vermischt 90 Gewichtsteile der Verbindung des Beispiels 1 mit 10 Gewichtsteilen N-Methyl-alpha-pyrrolidon und erhält eine Lösung, die zur Anwendung in Form kleinster Tropfen geeignet ist.

Beispiel II

10 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel III

20 Gewichtsteile der Verbindung des Beispiels 2 werden in einer Mischung gelöst, die aus 40 Gewichtsteilen Cyclohexanon, 30 Gewichtsteilen Isobutanol, 20 Gewichtsteilen

des Anlagerungsproduktes von 7 Mol Ethylenoxid an 1 Mol Isooctylphenol und 10 Gewichteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel IV

20 Gewichtsteile der Verbindung des Beispiels 2 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel V

20 Gewichtsteile des Wirkstoffs gemäß Beispiel 1 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel VI

95 Gewichtsteile der Verbindung des Beispiels 1 werden mit 5 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs des Beispiels 1 werden mit 10 Teilen Natriumsalz eines Phenolsulfonsäure-harnstoff--formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel IX

20 Teile des Wirkstoffs des Beispiels 1 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Applikation kann im Vorauflaufverfahren oder bei Nachauflaufanwendung erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr.

Der Einfluß der 2-Phenyl-1,3-dioxane der Formel I auf das Wachstum von erwünschten und unerwünschten Pflanzen wird anhand von Gewächshausversuchen vorgeführt:

Als Kulturgefäße dienten Plastikblumentöpfe mit 300 $cm^3$ Inhalt und lehmiger Sand mit etwa 1,5 % Humus als Substrat. Bei Soja und Reis wurde zur Verbesserung des Wachstums etwas Torfmull (peat) zugemischt. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 2,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung

bewirkte ein gleichmäßiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach. Die Aufwandmengen für die Nachauflaufbehandlung betrug 4,0 kg/ha Wirkstoff. Eine Abdeckung unterblieb bei der Nachauflaufbehandlung. Die Aufstellung der Versuche erfolgte im Gewächshaus, wobei für wärmeliebende Arten wärmere Bereiche (20 bis 40°C) und für solche gemäßigter Klimate 10 bis 20°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung und normaler Ablauf und 100 kein Aufgang der Pflanzen bzw. völlige Zerstörung zumindest der oberirdischen Teile.

Die Gewächshausversuche ergeben, daß die Verbindung Nr. 1 bei Vorauflaufanwendung von 2,0 kg Wirkstoff/ha eine gute Wirkung gegen unerwünschte Pflanzen aus der Familie der Gramineen (Gräser) besitzt und gleichzeitig für wichtige landwirtschaftliche Kulturen verträglich ist.

Ebenso bekämpft in diesen Gewächshausversuchen die Verbindung Nr. 2 bei Nachauflaufanwendung von 4,0 kg Wirkstoff/ha unerwünschte Grasarten, während Kulturpflanzen ungeschädigt bleiben.

In Anbetracht der guten Verträglichkeit der Wirkstoffe und der Vielseitigkeit der Applikationsmethoden können die Verbindungen der Formel I oder diese enthaltende Mittel außer bei den bereits aufgeführten Nutzpflanzen noch in

einer weiteren großen Zahl von Kulturpflanzen zur Beseitigung unerwünschten Pflanzenwuchses eingesetzt werden.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht phytotoxische Öle und Ölkonzentrate zugesetzt werden.

Patentansprüche

1. Herbizides Mittel, enthaltend eine Verbindung der Formel I

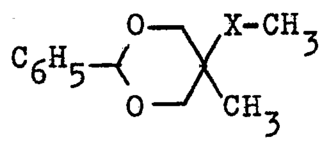

I,

worin X eine CO- oder CHOH-Gruppe darstellt.

2. Herbizides Mittel, enthaltend eine Verbindung der Formel I

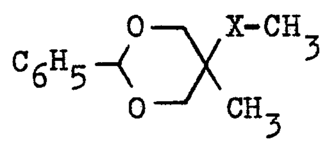

I,

worin X eine CO- oder CHOH-Gruppe darstellt und einen festen oder flüssigen Trägerstoff.

3. Verwendung der Verbindung der Formel I gemäß Anspruch 1 zur Bekämpfung unerwünschten Pflanzenwuchses.

4. Verfahren zur Bekämpfung unerwünschten Pflanzenwuchses, dadurch gekennzeichnet, daß man die Pflanzen oder den Boden mit einer Verbindung gemäß Anspruch 1 behandelt.

5. Verbindung der Formel I gemäß Anspruch 1 zur Verwendung bei der Bekämpfung unerwünschten Pflanzenwuchses.

Europäisches Patentamt

# EUROPÄISCHER RECHERCHENBERICHT

0065196

Nummer der Anmeldung

EP 82 10 3841

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-3 792 062 (E.G.TEACH) *Spalten 1-9* | 1-5 | A 01 N 43/32 |
| | --- | | |
| D,A | TETRAHEDRON, Band 26, 1970, Seiten 693-699, (GB) T.A.CRABB et al.: "The NMR spectra and configurations of some 2,5,5-Trisubstituted 1,3-Dioxans". *Seiten 693,694,696,698* | 1 | |
| | ----- | | |
| | | | **RECHERCHIERTE SACHGEBIETE (Int. Cl. 3)** |
| | | | A 01 N 43/00<br>C 07 D 319/06 |

Der vorliegende Recherchenbericht wurde für alle Patentansprüche erstellt.

| Recherchenort | Abschlußdatum der Recherche | Prüfer |
|---|---|---|
| DEN HAAG | 20-08-1982 | FRANCOIS J.C.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument